# EUROPEAN PATENT SPECIFICATION

(11) **EP 3 072 685 B1**
(45) Date of publication and mention of the grant of the patent: **10.04.2024**
(21) Application number: 15836841.5
(22) Date of filing: 18.08.2015
(51) Int. Cl.: B32B 25/10, C09J 123/00, A61F 13/49, B32B 5/02, B32B 7/12, B32B 27/32

(54) **STRETCHABLE LAMINATE, AND ARTICLE INCLUDING SAME**
DEHNBARES LAMINAT UND ARTIKEL DAMIT
STRATIFIÉ EXTENSIBLE, ET ARTICLE CONTENANT CELUI-CI

(30) Priority: 26.08.2014 JP 2014171312; 26.06.2015 JP 2015128993; 17.08.2015 JP 2015160253
(43) Date of publication of application: 28.09.2016
(73) Proprietor: Nitto Denko Corporation, Ibaraki-shi, Osaka 567-8680 (JP)
(72) Inventor: IKISHIMA, Shinsuke, Ibaraki-shi Osaka 567-8680 (JP); UCHIDA, Shou, Ibaraki-shi Osaka 567-8680 (JP); KONDOU, Hiroyuki, Ibaraki-shi Osaka 567-8680 (JP)
(74) Representative: Müller-Boré & Partner Patentanwälte PartG mbB
(86) International application number: PCT/JP2015/073104
(87) International publication number: WO 2016/031622

(56) References cited:
- EP-A1- 3 023 247
- JP-A- 2003 533 551
- JP-A- 2006 515 893
- JP-A- 2011 514 178
- JP-A- 2013 500 359
- US-A1- 2005 054 779
- US-A1- 2007 088 116
- US-A1- 2010 262 103
- US-A1- 2011 021 103
- US-A1- 2014 134 435

## Description

The present invention relates to a stretchable laminate and an article including the stretchable laminate.

Various stretchable laminates are proposed for materials for articles such as sanitary articles, for example, diapers and masks (see, for example, Patent Literatures 1 and 2) . Patent Literature 3 discloses a combination of elastomeric film composition and adhesive for a stretch laminate. Patent Literature 4 describes a hot melt adhesive based on olef in block copolymers . Patent Literature 5 discloses stretchable laminates of nonwoven webs and elastic film. Patent Literature 6 describes a low application temperature hot melt adhesive. Patent Literature 7 discloses a stretchable hot-melt adhesive composition with temperature resistance. Patent Literature 8 describes a stretchable laminate and article containing same.

As such materials, a stretchable laminate including an elastomer layer and a non-woven fabric layer arranged on at least one side of the elastomer layer has been proposed. In such stretchable laminate, the elastomer layer and the non-woven fabric layer are generally bonded onto each other with an adhesive or a pressure-sensitive adhesive.

However, in the related-art stretchable laminate including an elastomer layer and a non-woven fabric layer arranged on at least one side of the elastomer layer, there is a problem in that interlayer delamination may occur between the elastomer layer and the non-woven fabric layer when a load is applied onto a surface, on which the elastomer layer and the non-woven fabric layer are bonded onto each other, in a substantially perpendicular direction.

### Patent Literature

[PTL 1] JP 2012-187857 A
[PTL 2] JP 3830818 B2
[PTL 3] US 2014/134435 A1
[PTL 4] US 2011/021103 A1
[PTL 5] US 2010/262103 A1
[PTL 6] US 2007/088116 A1
[PTL 7] US 2005/054779 A1
[PTL 8] EP 3 023 247 A1

The present invention has been made to solve the problem of the related art, and an object of the present invention is to provide a stretchable laminate including an elastomer layer and a non-woven fabric layer arranged on at least one side of the elastomer layer, in which interlayer delamination does not occur between the elastomer layer and the non-woven fabric layer even when a load is applied onto a surface, on which the elastomer layer and the non-woven fabric layer are bonded onto each other, in a substantially perpendicular direction. Another object of the present invention is to provide an article including such stretchable laminate.

The present invention concerns a stretchable laminate as defined by claim 1 and an article comprising the laminate, as defined by claim 7. Further embodiments are defined by claims 2-6.

According to the present invention, the stretchable laminate including an elastomer layer and a non-woven fabric layer arranged on at least one side of the elastomer layer, in which interlayer delamination does not occur between the elastomer layer and the non-woven fabric layer even when a load is applied onto a surface, on which the elastomer layer and the non-woven fabric layer are bonded onto each other, in a substantially perpendicular direction can be provided. Further, the article including such stretchable laminate can be provided.

FIG. 1 is a schematic cross-sectional view of a stretchable laminate according to a preferred embodiment of the present invention.
FIG. 2 is a schematic view for illustrating another stretchable laminate according to a preferred embodiment of the present invention.
FIG. 3 is a schematic view of a state in which a non-woven fabric is coated with a hot-melt pressure-sensitive adhesive in a striped manner in a flow direction of a production line as viewed from a top surface thereof.

### <<<<Stretchable Laminate>>>>

A stretchable laminate of the present invention is a stretchable laminate including a non-woven fabric layer on at least one side of an elastomer layer. The stretchable laminate of the present invention may include any appropriate other layer to the extent that the effects of the present invention are not impaired as long as the stretchable laminate includes a non-woven fabric layer on at least one side of the elastomer layer. The number of such any appropriate other layers may be only one, or may be two or more.

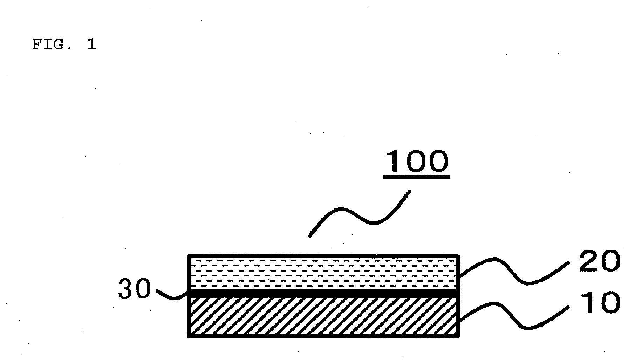

FIG. **1** is a schematic sectional view of a stretchable laminate according to a preferred embodiment of the present invention. A stretchable laminate **100** illustrated in FIG. **1** includes an elastomer layer **10** and a non-woven fabric layer **20** arranged only on one side of the elastomer layer **10.** The stretchable laminate **100** illustrated in FIG. **1** includes a hot-melt pressure-sensitive adhesive **30** between the elastomer layer **10** and the non-woven fabric layer **20.** In FIG. **1****,** the hot-melt pressure-sensitive adhesive **30** is illustrated so as to be present in the whole region between the elastomer layer 10 and the non-woven fabric layer **20,** but the hot-melt pressure-sensitive adhesive **30** may be present in part of a region between the elastomer layer **10** and the non-woven fabric layer **20.**

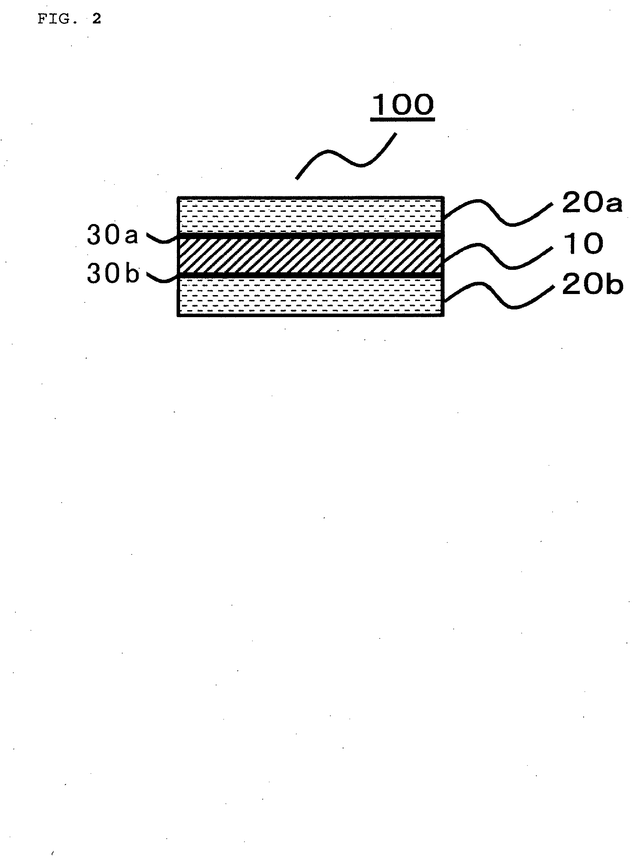

FIG. **2** is a schematic cross-sectional view of another stretchable laminate according to a preferred embodiment of the present invention. A stretchable laminate **100** illustrated in FIG. **2** includes the elastomer layer **10,** a non-woven fabric layer **20a** arranged on one side of the elastomer layer **10,** and a non-woven fabric layer **20b** arranged on the elastomer layer **10** on an opposite side to the non-woven fabric layer **20a.** The stretchable laminate **100** illustrated in FIG. **2** includes a hot-melt pressure-sensitive adhesive **30a** between the elastomer layer **10** and the non-woven fabric layer **20a,** and includes a hot-melt pressure-sensitive adhesive 30b between the elastomer layer 10 and the non-woven fabric layer **20b.** In FIG. **2****,** the hot-melt pressure-sensitive adhesive **30a** is illustrated so as to be present in the whole region between the elastomer layer **10** and the non-woven fabric layer **20a,** but the hot-melt pressure-sensitive adhesive **30a** may be present in part of a region between the elastomer layer **10** and the non-woven fabric layer **20a.** Further, similarly, in FIG. **2****,** the hot-melt pressure-sensitive adhesive **30b** is illustrated so as to be present in the whole region between the elastomer layer **10** and the non-woven fabric layer **20b,** but the hot-melt pressure-sensitive adhesive **30b** may be present in part of a region between the elastomer layer **10** and the non-woven fabric layer **20b.**

The thickness of the stretchable laminate of the present invention varies depending on the thickness of the elastomer layer or the thickness of the non-woven fabric layer and is preferably from 1.0 mm to 0.1 mm, more preferably from 0.8 mm to 0.15 mm, still more preferably from 0.6 mm to 0.15 mm, particularly preferably from 0.5 mm to 0.2 mm, most preferably from 0.45 mm to 0.2 mm. When the thickness of the stretchable laminate of the present invention falls within such range, the laminate can be easily used as a material used in articles such as sanitary articles, for example, diapers and masks.

### <<Elastomer Layer>>

As the elastomer layer, any appropriate elastomer layer may be adopted to the extent that the effects of the present invention are not impaired. As an elastomer resin serving as a main component in such elastomer layer, there are given, for example, an olefin-based elastomer, a styrene-based elastomer, a vinyl chloride-based elastomer, a urethane-based elastomer, an ester-based elastomer, and an amide-based elastomer. According to the present invention, the elastomer layer comprises a propylene-based elastomer as a main component.

The content of the elastomer resin serving as a main component in the elastomer layer is from 50 wt% to 100 wt%, more preferably from 70 wt% to 100 wt%, still more preferably from 90 wt% to 100 wt%, particularly preferably from 95 wt% to 100 wt%, most preferably from 98 wt% to 100 wt%. When the content of the elastomer resin serving as a main component in the elastomer layer falls within the range described above, the elastomer layer can exhibit a sufficient elastomer characteristic.

The number of the elastomer layers may be one, or may be two or more. When the elastomer layer has a three-layer structure, for example, it is preferred that the three-layer structure include an intermediate layer in which two or more kinds of elastomers are blended and two surface layers in which the same kinds of elastomers as the elastomers included in the intermediate layer are used.

In the present invention, the elastomer resin serving as a main component in the elastomer layer is a propylene-based elastomer. In the following, the propylene-based elastomer is also referred to as the olefin-based elastomer or α-olefin-based elastomer. In the invention the elastomer is propylene-based. When the olefin-based elastomer is adopted as the elastomer resin, the heat stability is enhanced compared to any other elastomer resin (for example, a styrene-based elastomer), and hence, for example, heat deterioration during film formation in producing the stretchable laminate of the present invention can be suppressed. In addition, when the olefin-based elastomer is adopted as the elastomer resin, the storage stability is enhanced compared to any other elastomer resin (for example, a styrene-based elastomer), and hence fluctuation of values for physical properties during storage of the stretchable laminate of the present invention can be suppressed.

In the present invention, as the olefin-based elastomer is adopted as the elastomer resin, the steps in production of the elastomer layer can be simplified, and hence processing cost can be reduced. This is, for example, because when the other elastomer resin (for example, a styrene-based elastomer) is adopted as the elastomer resin, several kinds of styrene-based elastomers need to be blended with one another in order to control values for physical properties, which needs to prepare a master batch. When the olefin-based elastomer is adopted as the elastomer resin, extrusion molding can be performed by using fewer kinds of resins in production of the elastomer layer, which can obviate the need to prepare a master batch.

In the present invention, as the olefin-based elastomer is adopted as the elastomer resin, one kind of the olefin-based elastomers may be used alone, or two or more kinds thereof may be used as a blend.

Examples of the olefin-based elastomer include an olefin block copolymer, an olefin random copolymer, an ethylene copolymer, a propylene copolymer, an ethylene olefin block copolymer, a propylene olefin block copolymer, an ethylene olefin random copolymer, a propylene olefin random copolymer, an ethylene propylene random copolymer, an ethylene (1-butene) random copolymer, an ethylene (1-pentene) olefin block copolymer, an ethylene (1-hexene) random copolymer, an ethylene (1-heptene) olefin block copolymer, an ethylene (1-octene) olefin block copolymer, an ethylene (1-nonene) olefin block copolymer, an ethylene (1-decene) olefin block copolymer, a propylene ethylene olefin block copolymer, an ethylene (a-olefin) copolymer, an ethylene (a-olefin) random copolymer, an ethylene (a-olefin) block copolymer, and combinations thereof. According to the present invention, the olefin-based elastomer is a propylene-based elastomer.

The propylene-based elastomer resin has a density of preferably from 0.890 g/cm³ to 0.830 g/cm³, more preferably from 0.888 g/cm³ to 0.835 g/cm³, still more preferably from 0.886 g/cm³ to 0.835 g/cm³, particularly preferably from 0.885 g/cm³ to 0.840 g/cm³, most preferably from 0.885 g/cm³ to 0.845 g/cm³. When the olefin-based elastomer whose density falls within the range described above is adopted, a stretchable laminate having more excellent fittability can be provided, and the heat stability is further enhanced compared to any other elastomer resin (for example, a styrene-based elastomer), and hence, for example, heat deterioration during film formation in producing the stretchable laminate of the present invention can be further suppressed. Inaddition, the storage stability is further enhanced compared to any other elastomer resin (for example, a styrene-based elastomer), and hence fluctuation of values for physical properties during storage of the stretchable laminate of the present invention can be further suppressed. Further, the steps in production of the elastomer layer can be further simplified, and hence processing cost can be further reduced.

In the present invention, the propylene-based elastomer resin has a MFR at 230°C and 2.16 kgf of preferably from 1.0 g/10 min to 25.0 g/10 min, more preferably from 2.0 g/10 min to 23.0 g/10 min, still more preferably from 2.0 g/10 min to 21.0 g/10 min, particularly preferably from 2.0 g/10 min to 20.0 g/10 min, most preferably from 2.0 g/10 min to 19.0 g/10 min. When the olefin-based elastomer whose MFR falls within the range described above is adopted, a stretchable laminate having more excellent fittability can be provided, and the heat stability is further enhanced compared to any other elastomer resin (for example, a styrene-based elastomer), and hence, for example, heat deterioration during film formation in producing the stretchable laminate of the present invention can be further suppressed. In addition, the storage stability is further enhanced compared to any other elastomer resin (for example, a styrene-based elastomer), and hence fluctuation of values for physical properties during storage of the stretchable laminate of the present invention can be further suppressed. Further, the steps in production of the elastomer layer can be further simplified, and hence processing cost can be further reduced.

In the present invention, the propylene-based elastomer resin is an α-olefin-based elastomer. That is, the α-olefin-based elastomer is a copolymer of two or more kinds of α-olefins and has elastomer characteristics. Among such α-olefin-based elastomers, in general, at least one kind selected from an ethylene-based elastomer, a propylene-based elastomer, and a 1-butene-based elastomer is more preferred. According to the present invention, the α-olefin-based elastomer is a propylene-based elastomer. When such α-olefin-based elastomer is adopted as the olefin-based elastomer, a stretchable laminate having more excellent fittability can be provided, and the heat stability is further enhanced compared to any other elastomer resin (for example, a styrene-based elastomer), and hence, for example, heat deterioration during film formation in producing the stretchable laminate of the present invention can be further suppressed. In addition, the storage stability is further enhanced compared to any other elastomer resin (for example, a styrene-based elastomer), and hence fluctuation of values for physical properties during storage of the stretchable laminate of the present invention can be further suppressed. Further, the steps in production of the elastomer layer can be further simplified, and hence processing cost can be further reduced.

In the present invention, as the propylene-based elastomer is adopted as the olefin-based elastomer, a stretchable laminate having extremely excellent fittability can be provided, and the heat stability is further enhanced compared to any other elastomer resin (for example, a styrene-based elastomer), and hence, for example, heat deterioration during film formation in producing the stretchable laminate of the present invention can be further suppressed. Inaddition, the storage stability is further enhanced compared to any other elastomer resin (for example, a styrene-based elastomer), and hence fluctuation of values for physical properties during storage of the stretchable laminate of the present invention can be further suppressed. Further, the steps in production of the elastomer layer can be further simplified, and hence processing cost can be further reduced.

The propylene-based elastomer as described above is also available as a commercial product. Examples of such commercial product include some products in the "TAFMER" (trademark) series (such as TAFMER PN-3560) manufactured by Mitsui Chemicals, Inc., and some products in the "Vistamaxx" (trademark) series (such as Vistamaxx 3000, Vistamaxx 6202, and Vistamaxx 7010) manufactured by Exxon Mobil Corporation.

In the present invention, the propylene-based elastomer resin is preferably produced by using a metallocene catalyst. In the α-olefin-based elastomer produced by using a metallocene catalyst, a stretchable laminate having extremely excellent fittability can be provided, and the heat stability is further enhanced compared to any other elastomer resin (for example, a styrene-based elastomer), and hence, for example, heat deterioration during film formation in producing the stretchable laminate of the present invention can be further suppressed. In addition, the storage stability is further enhanced compared to any other elastomer resin (for example, a styrene-based elastomer), and hence fluctuation of values for physical properties during storage of the stretchable laminate of the present invention can be further suppressed. Further, the steps in production of the elastomer layer can be further simplified, and hence processing cost can be further reduced.

The elastomer layer may contain any appropriate other component to the extent that the effects of the present invention are not impaired. Examples of such other component include any other polymer, a tackifier, a plasticizer, an antidegradant, a pigment, a dye, an antioxidant, an antistatic agent, a lubricant, a blowing agent, a heat stabilizer, a light stabilizer, an inorganic filler, and an organic filler. The number of kinds of those components may be only one, or may be two or more. The content of the other component in the elastomer layer is preferably 10 wt% or less, more preferably 7 wt% or less, still more preferably 5 wt% or less, particularly preferably 2 wt% or less, most preferably 1 wt% or less.

The thickness of the elastomer layer is preferably from 200 µm to20 µm, more preferably from 160 µm to 30 µm, still more preferably from 140 µm to 30 µm, particularly preferably from 120 µm to 30 µm, most preferably from 100 µm to 30 µm. When the thickness of the elastomer layer falls within such range, a stretchable laminate having more excellent fittability can be provided.

### <<Non-woven Fabric Layer>>

Any appropriate non-woven fabric layer may be adopted as the non-woven fabric layer to the extent that the effects of the present invention are not impaired. The number of kinds of non-woven fabrics forming the non-woven fabric layer may be only one, or may be two or more.

Examples of the non-woven fabric forming the non-woven fabric layer include a spunbond non-woven web, a raised non-woven fabric (such as a non-woven fabric obtained by a thermal bonding method, a bonding joining method, or a spunlace method), a melt-blown non-woven web, a spunlace non-woven web, a spunbond melt-blown spunbond non-woven web, a spunbond melt-blown melt-blown spunbond non-woven web, an unjoined non-woven web, an electrospun non-woven web, a flashspun non-woven web (such as TYVEKTM of DuPont), and a carded non-woven fabric.

The non-woven fabric forming the non-woven fabric layer contains polyolefin fibers, the polyolefin being polypropylene. The non-woven fabric forming the non-woven fabric layer may further contain polyolefin fibers, such as polyethylene, polyester, polyamide, polyurethane, an elastomer, rayon, cellulose, acryl, copolymers thereof, blends thereof, or mixtures thereof.

The non-woven fabric forming the non-woven fabric layer may contain a fiber which has a uniform structure and may contain a two-component structure, such as a sheath/core structure, a side-by-side structure, a sea-island structure, or any other two-component structure. Detailed descriptions of the non-woven fabric can be found in, for example, "Nonwoven Fabric Primer and Reference Sampler, " E.A. Vaughn, Association of the Nonwoven Fabrics Industry, third edition (1992).

The basis amount of the non-woven fabric forming the non-woven fabric layer is preferably 150 gsm or less, more preferably 100 gsm or less, still more preferably 50 gsm or less, particularly preferably from 10 gsm to 30 gsm.

### <<Hot-melt Pressure-sensitive Adhesive>>

The stretchable laminate of the present invention includes the hot-melt pressure-sensitive adhesive between the elastomer layer and the non-woven fabric layer. When the hot-melt pressure-sensitive adhesive is used, the need to add a tackifier as a component of the elastomer layer is reduced, and hence, for example, the extrusion stability is enhanced, a problem of adhesion of the tackifier to a forming roll can be suppressed, and a problem of contamination of the production line by volatile matter contamination or the like caused by the tackifier can be suppressed.

The hot-melt pressure-sensitive adhesive is used for bonding the elastomer layer and the non-woven fabric layer, the hot-melt pressure-sensitive adhesive may be present over the whole surface of the non-woven fabric layer or may be present in part of the surface of the non-woven fabric layer. When the hot-melt pressure-sensitive adhesive is present in part of the surface of the non-woven fabric layer, the hot-melt pressure-sensitive adhesive is preferably present so as to include at least an end portion of the non-woven fabric layer.

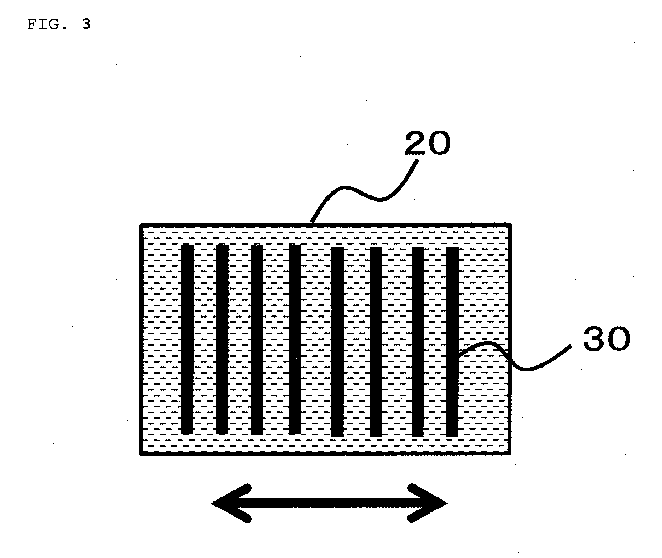

The hot-melt pressure-sensitive adhesive is used for bonding the elastomer layer and the non-woven fabric layer, for example, as illustrated in FIG. **3****,** the hot-melt pressure-sensitive adhesive **30** may be present in a striped manner or in a dotted manner on the non-woven fabric layer **20** in a flow direction of the production line. When the hot-melt pressure-sensitive adhesive is present in a striped manner, portions in which the hot-melt pressure-sensitive adhesive is present and portions in which the hot-melt pressure-sensitive adhesive is absent are formed in a striped manner, and therefore, particularly in a direction perpendicular to the stripe pattern (in the direction of the arrow in FIG. 3), the stretchability of the stretchable laminate can be further enhanced.

The hot-melt pressure-sensitive adhesive contains an olefin component. When the hot-melt pressure-sensitive adhesive contains the olefin component, a stretchable laminate including an elastomer layer and a non-woven fabric layer arranged on at least one side of the elastomer layer, in which interlayer delamination does not occur between the elastomer layer and the non-woven fabric layer even in the case where a load is applied onto a surface, on which the elastomer layer and the non-woven fabric layer are bonded onto each other, in a substantially perpendicular direction, can be provided.

The number of kinds of the olefin components in the hot-melt pressure-sensitive adhesive may be only one, or may be two or more.

The hot-melt pressure-sensitive adhesive may contain any appropriate other component to the extent that the effects of the present invention are not impaired as long as the hot-melt pressure-sensitive adhesive contains an olefin component. Examples of such other component include liquid paraffin, a tackifier, an antioxidant, a UV absorber, a light stabilizer, and a fluorescence agent. Among such other components, at least one kind selected from liquid paraffin, a tackifier, and an antioxidant is preferably contained in the hot-melt pressure-sensitive adhesive from the viewpoint of enabling the effects of the present invention to be further exhibited. The number of kinds of such other components may be only one, or may be two or more.

The tackifier is effective for improving a pressure-sensitive adhesive strength. When the hot-melt pressure-sensitive adhesive contains the tackifier, the content of the tackifier in the hot-melt pressure-sensitive adhesive is from 35 wt% to 55 wt% from the viewpoint of enabling the effects of the present invention to be further exhibited.

Examples of the tackifier include a hydrocarbon-based tackifier, a terpene-based tackifier, a rosin-based tackifier, a phenol-based tackifier, an epoxy-based tackifier, a polyamide-based tackifier, an elastomer-based tackifier, and a ketone-based tackifier. The number of kinds of the tackifiers may be only one, or may be two or more.

Examples of the hydrocarbon-based tackifier include an aliphatic hydrocarbon resin, an aromatic hydrocarbon resin (such as a xylene resin), an aliphatic cyclic hydrocarbon resin, an aliphatic/aromatic petroleum resin (such as a styrene-olefin-based copolymer), an aliphatic/alicyclic petroleum resin, a hydrogenated hydrocarbon resin, a coumarone-based resin, and a coumarone-indene-based resin.

Examples of the terpene-based tackifier include: terpene-based resins, such as an α-pinene polymer and a β-pinene polymer; and modified terpene-based resins (such as a terpene-phenol-based resin, a styrene-modified terpene-based resin, and a hydrogenated terpene-based resin) each obtained by subj ecting a terpene-based resin to modification (such as phenol modification, aromatic modification, or hydrogenation modification).

Examples of the rosin-based tackifier include: unmodified rosins (raw rosins), such as gum rosin and wood rosin; modified rosins (such as a hydrogenated rosin, a disproportionated rosin, a polymerized rosin, and any other chemically modified rosin) each obtained by subjecting an unmodified rosin to modification, such as hydrogenation, disproportionation, or polymerization; and other various rosin derivatives.

An example of the phenol-based tackifier is a resol-type or novolac-type alkylphenol.

The tackifier may be a product commercially available as a blend product of an olefin resin and a thermoplastic elastomer.

According to the present invention, the olefin component contained in the hot-melt pressure-sensitive adhesive comprises an olefin-based polymer. Such olefin-based polymer is also available as a commercial product. General examples of commercial product include some products in the "VISTAMAXX" (trademark) series (such as VISTAMAXX 6202, VISTAMAXX 7010, and VISTAMAXX 7050) manufactured by ExxonMobil Corporation and some products in the "REXtac" (trademark) series (such as REXTAC RT2788) manufactured by REXtac, LLC.

The hot-melt pressure-sensitive adhesive contains an olefin-based polymer, the number of kinds of the olefin-based polymers may be only one, or may be two or more.

According to the present invention, the olefin-based polymer comprises a 1-butene copolymer. Examples of the olefin-based polymer further include an olefin-based elastomer, and an ethylene-propylene copolymer. The olefin-based polymer is a 1-butene copolymer from the viewpoint of enabling the effects of the present invention to be further exhibited. That is, the olefin-based polymer includes a 1-butene copolymer as an essential component and may further include an olefin-based elastomer. The content of the 1-butene copolymer in the hot-melt pressure-sensitive adhesive is from 35 wt% to 60 wt% from the viewpoint of enabling the effects of the present invention to be further exhibited.

As the olefin-based elastomer, any appropriate olefin-based elastomer may be adopted to the extent that the effects of the present invention are not impaired. Examples of such olefin-based elastomer include an α-olefin-based elastomer, a propylene-1-butene copolymer, and an ethylene-propylene copolymer. Of those, from the viewpoint of enabling the effects of the present invention to be further exhibited, an α-olefin-based elastomer is preferred. The number of kinds of the olefin-based elastomers may be only one, or may be two or more.

A preferred example of the α-olefin-based elastomer is at least one kind selected from an ethylene-based elastomer, a propylene-based elastomer, and a 1-butene-based elastomer. Of those, a propylene-based elastomer is preferred from the viewpoint of enabling the effects of the present invention to be further exhibited. That is, it is preferred that the α-olefin-based elastomer include at least one kind selected from an ethylene-based elastomer, a propylene-based elastomer, and a 1-butene-based elastomer, and it is more preferred that the α-olef in-based elastomer include a propylene-based elastomer as an essential component and may further include an ethylene-based elastomer or a 1-butene-based elastomer.

The α-olefin-based elastomer is also available as a commercial product. Examples of such commercial product include some products in the "TAFMER" (trademark) series (such as TAFMER PN-3560) manufactured by Mitsui Chemicals, Inc. and some products in the "VISTAMAXX" (trademark) series (such as VISTAMAXX 3000, VISTAMAXX 6202, VISTAMAXX 7010, and VISTAMAXX 7050) manufactured by ExxonMobil Corporation.

As the 1-butene copolymer, any appropriate 1-butene copolymer may be adopted to the extent that the effects of the present invention are not impaired. Examples of such 1-butene copolymer include an ethylene/1-butene copolymer and a propylene/1-butene copolymer. Of those, a propylene/1-butene copolymer is preferred from the viewpoint of enabling the effects of the present invention to be further exhibited. The number of kinds of the 1-butene copolymers may be only one, or may be two or more.

The 1-butene copolymer is also available as a commercial product. Examples of such commercial product include some products in the "Rextac" (trademark) series (such as REXTAC RT2788) manufactured by REXtac, LLC.

The hot-melt pressure-sensitive adhesive in the stretchable laminate of the present invention has a non-woven fabric adhesive strength described later (adhesive strength with respect to a spunbond non-woven fabric for a diaper back sheet made of 100% polypropylene (19 g/m²) at an angle of 90° and a peel rate of 300 mm/min) of preferably 190 g/25 mm or more, more preferably 220 g/25 mm or more, still more preferably 240 g/25 mm or more, yet still more preferably 260 g/25 mm or more, even yet still more preferably 280 g/25 mm or more, even yet still more preferably 300 g/25 mm or more, even yet still more preferably 320 g/25 mm or more, particularly preferably 340 g/25 mm or more, most preferably 350 g/25 mm or more. The upper limit value of the non-woven fabric adhesive strength is preferably 10,000 g/25 mm, more preferably 5, 000 g/25 mm, still more preferably 1,000 g/25 mm, yet still more preferably 800 g/25 mm, particularly preferably 600 g/25 mm, most preferably 500 g/25 mm. When the non-woven fabric adhesive strength is adjusted within the range described above, a stretchable laminate including an elastomer layer and a non-woven fabric layer arranged on at least one side of the elastomer layer, in which interlayer delamination does not occur between the elastomer layer and the non-woven fabric layer even when a load is applied onto a surface, on which the elastomer layer and the non-woven fabric layer are bonded onto each other, in a substantially perpendicular direction, can be provided.

### <<Production of Stretchable Laminate of the Present Invention>>

In production of the stretchable laminate of the present invention, the elastomer layer and the non-woven fabric layer are directly laminated with each other (for example, FIG. **1** and FIG. **2**), and hence the elastomer layer and the non-woven fabric layer need to be bonded onto each other. Examples of such bonding method include: (1) a method involving laminating an elastomer layer formed by extrusion molding from a T-die of an extruder and a non-woven fabric layer separately fed from a rolled body; (2) a method involving laminating an elastomer layer and a non-woven fabric layer by co-extrusion; (3) a method involving bonding an elastomer layer and a non-woven fabric layer, which are prepared separately, with an adhesive; (4) a method involving forming a non-woven fabric layer on an elastomer layer, which has been formed by any appropriate method, by a melt-blown method or the like; and (5) thermally laminating or ultrasonically welding an elastomer layer and a non-woven fabric layer.

In production of the stretchable laminate of the present invention, the hot-melt pressure-sensitive adhesive is preferably used for bonding the elastomer layer and the non-woven fabric layer onto each other. For example, in the case of applying the method (1) described above, the non-woven fabric layer separately fed from a rolled body may be coated with the hot-melt pressure-sensitive adhesive before being laminated with the elastomer layer.

The stretchable laminate of the present invention can be subjected to treatments referred to as pre-extension treatment and activation treatment after laminating the elastomer layer and the non-woven fabric layer. Specifically, extension treatment is performed in a width direction of the stretchable laminate or treatment in which a fiber structure of part of the region of the non-woven fabric layer is mechanically broken can be performed. When such treatments are performed, the stretchable laminate can be elongated by a smaller force.

### <<Application of Stretchable Laminate of the Present Invention>>

The stretchable laminate of the present invention can be used in any appropriate article in which the effects of the present invention can be effectively utilized. That is, the article of the present invention includes the stretchable laminate of the present invention. A typical example of such article is a sanitary article. Examples of such sanitary article include a diaper (particularly an ear portion of a disposable diaper), a supporter, and a mask. Examples

The present invention is hereinafter specifically described by way of Examples. Examples 1 - 6 are examples of the invention. Examples 7 and 8 are reference examples, not being examples of the invention.

However, the present invention is by no means limited by these Examples. In Examples and the like, test and evaluation methods are as described below. In addition, "part (s) " means "part (s) by weight" and "%" means "wt%" unless otherwise stated.

### <Evaluation of Non-woven Fabric Adhesive Strengths

A sheet obtained by applying a prepared hot-melt pressure-sensitive adhesive onto an OPP film (35 µm) at 15 g/m² was cut into a size of 25 mm in width, and the resultant sheet was compressively bonded onto a spunbond non-woven fabric for a diaper back sheet made of 100% polypropylene (19 g/m²) by two reciprocations under a load of 1 kg. The resultant was left to stand still at room temperature for 10 minutes after the compressive bonding, and an adhesive strength was measured at an angle of 90° and a peel rate of 300 mm/min.

### <40°C Retention Ability Test (Evaluation of Presence/absence of Delamination)>

667 parts of a propylene/1-butene copolymer (manufactured by REXtac, LLC, trade name: REXTAC RT2788), 520 parts of a tackifier (manufactured by Kolon Industries, Inc., trade name: SUKOREZ SU-100S), 100 parts of liquid paraffin (manufactured by Petro yag, trade name: White Oil Pharma Oyster 259), and 13 parts of an antioxidant (manufactured by BASF Japan Co., Ltd., trade name: Irganox 1010) were blended to provide a hot-melt pressure-sensitive adhesive. A sheet obtained by applying the hot-melt pressure-sensitive adhesive onto an OPP film (35 µm) at 15 g/m² was cut into a size of 25 mm in width. The resultant sheet was bonded onto each of the non-woven fabric surfaces (one surface side, whole surface applied portion) of the stretchable laminates produced in Examples and Comparative Examples with a width of 25 mm and a length of 15 mm and compressively bonded thereonto by two reciprocations under a load of 1 kg. The resultant was left to stand still at room temperature for 10 minutes after the compressive bonding, and then left to stand still for 30 minutes under an environment of 40°C. The resultant was cut into a size of 50 mm in width in a film CD direction and set on a retention ability testing machine so that a load of 1 kg was applied to the OPP film side. The case where a peeling (delamination) phenomenon occurred and the sheet fell after 2 hours was defined as NG, and the case where no delamination and falling occurred was defined as OK.

### <Molding Conditions>

In Examples and Comparative Examples, an elastomer layer (hereinafter sometimes referred to as elastic film) was formed by extrusion molding by extruding three layers in two types (A layer/B layer/A layer) through the use of a T-die molding machine. The extrusion temperatures were set under the following conditions.
A layer: 200°C
B layer: 200°C
Die temperature: 200°C

A non-woven fabric (PP carded type, basis amount=24 gsm) was bonded onto both surfaces of the elastic film extruded from the T-die through use of a roll to provide a stretchable laminate. In this case, a hot-melt pressure-sensitive adhesive was applied onto a bonded side of the non-woven fabric so as to alternately have a portion (A) in which the hot-melt pressure-sensitive adhesive had been applied onto the whole surface (7 g/m²) with a width of 30 mm and a portion (B) in which the hot-melt pressure-sensitive adhesive was applied in a striped manner (pressure-sensitive adhesive width: 1 mm, interval: 1 mm) (15 g/m²) with a width of 41 mm.

### [Example 1]

100 wt% of an olefin-based resin (manufactured by Exxon Mobil Corporation, trade name: Vistamaxx 6202) was loaded into A layer in an extrusion machine and a formulation of 65 wt% of an olefin-based resin (manufactured by Exxon Mobil Corporation, trade name: Vistamaxx 6202), 30 wt% of an olefin-based resin (manufactured by Mitsui Chemicals, Inc., trade name: TAFMER PN-3560), and 5 wt% of a white pigment (titanium oxide manufactured by Dupont, trade name: Ti-Pure R103) was loaded into B layer in the extrusion machine to extrude an elastic film (1) having the construction of A layer/B layer/A layer=6.75 µm/31.5 µm/6.75 um in total of 45 um.

Next, 667 parts of a propylene/1-butene copolymer (manufactured by REXtac, LLC, trade name: REXTAC RT2788), 520 parts of a tackifier (manufactured by Kolon Industries, Inc., trade name: SUKOREZ SU-100S), 100 parts of liquid paraffin (manufactured by Petro yag, trade name: White Oil Pharma Oyster 259), and 13 parts of an antioxidant (manufactured by BASF Japan Co., Ltd., trade name: Irganox 1010) were blended to provide a hot-melt pressure-sensitive adhesive (1).

The resultant elastic film (1) and hot-meltpressure-sensitive adhesive (1) were subjected to the above-mentioned <Forming Conditions>, and a portion (A) in which the hot-melt pressure-sensitive adhesive had been applied onto the whole surface (7 g/m²) with a width of 30 mm was cut at the middle to provide a stretchable laminate (1) having two portions (A1) and (A2), in which the hot-melt pressure-sensitive adhesive had been applied onto the whole surface (7 g/m²) with a width of 15 mm from both ends, and a portion (B), in which the hot-melt pressure-sensitive adhesive had been applied (15 g/m²) between the (A1) and the (A2) with a width of 41 mm in a striped manner (pressure-sensitive adhesive width: 1 mm, interval: 1 mm), on both surfaces of the elastic film.

The results are shown in Table 1 and Table 2.

### [Example 2]

An elastic film (2) was extruded in the same manner as in Example 1.

Next, 100 parts of a propylene-based elastomer (manufactured by Exxon Mobil Corporation, trade name: Vistamaxx 6202), 517 parts of a propylene/1-butene copolymer (manufactured by REXtac, LLC, trade name: REXTAC RT2788), 520 parts of a tackifier (manufactured by Exxon Mobil Corporation, trade name: Escorez 5400), 150 parts of liquid paraffin (manufactured by Petro yag, trade name: White Oil Pharma Oyster 259), and 13 parts of an antioxidant (manufactured by BASF Japan Co., Ltd., trade name: Irganox 1010) were blended to provide a hot-melt pressure-sensitive adhesive (2).

The resultant elastic film (2) and hot-meltpressure-sensitive adhesive (2) were subjected to the above-mentioned <Forming Conditions>, and a portion (A) in which the hot-melt pressure-sensitive adhesive had been applied onto the whole surface (7 g/m²) with a width of 30 mm was cut at the middle to provide a stretchable laminate (2) having two portions (A1) and (A2), in which the hot-melt pressure-sensitive adhesive had been applied onto the whole surface (7 g/m²) with a width of 15 mm from both ends, and a portion (B), in which the hot-melt pressure-sensitive adhesive had been applied (15 g/m²) between the (A1) and the (A2) with a width of 41 mm in a striped manner (pressure-sensitive adhesive width: 1 mm, interval: 1 mm), on both surfaces of the elastic film. The results are shown in Table 1 and Table 2.

### [Example 3]

A formulation of 50 wt% of an olefin-based resin (manufactured by Exxon Mobil Corporation, trade name: Vistamaxx 6202) and 50 wt% of an olefin-based resin (manufactured by Mitsui Chemicals, Inc. , trade name: TAFMER PN-3560, density=0.866 g/cm³, MFR=6.0 g/10 min) was loaded into A layer in an extrusion machine and a formulation of 45 wt% of an olefin-based resin (manufactured by Exxon Mobil Corporation, trade name: Vistamaxx 3000), 50 wt% of an olefin-based resin (manufactured by Mitsui Chemicals, Inc., trade name: TAFMER PN-3560), and 5 wt% of a white pigment (titanium oxide manufactured by Dupont, trade name: Ti-Pure R103) was loaded into B layer in the extrusion machine to extrude an elastic film (3) having the construction of A layer/B layer/A layer=6.75 µm/31.5 µm/6.75 um in total of 45 µm.

Next, 100 parts of a propylene-based elastomer (manufactured by Exxon Mobil Corporation, trade name: Vistamaxx 7050), 517 parts of a propylene/1-butene copolymer (manufactured by REXtac, LLC, trade name: REXTAC RT2730), 520 parts of a tackifier (manufactured by Exxon Mobil Corporation, trade name: Escorez 5400), 150 parts of liquid paraffin (manufactured by Petro yag, trade name: White Oil Pharma Oyster 259), and 13 parts of an antioxidant (manufactured by BASF Japan Co., Ltd., trade name: Irganox 1010) were blended to provide a hot-melt pressure-sensitive adhesive (3).

The resultant elastic film (3) and hot-melt pressure-sensitive adhesive (3) were subjected to the above-mentioned <Forming Conditions>, and a portion (A) in which the hot-melt pressure-sensitive adhesive had been applied onto the whole surface (7 g/m²) with a width of 30 mm was cut at the middle to provide a stretchable laminate (3) having two portions (A1) and (A2), in which the hot-melt pressure-sensitive adhesive had been applied onto the whole surface (7 g/m²) with a width of 15 mm from both ends, and a portion (B), in which the hot-melt pressure-sensitive adhesive had been applied (15 g/m²) between the (A1) and the (A2) with a width of 41 mm in a striped manner (pressure-sensitive adhesive width: 1 mm, interval: 1 mm), on both surfaces of the elastic film. The results are shown in Table 1 and Table 2.

### [Example 4]

A formulation of 50 wt% of an olefin-based resin (manufactured by Exxon Mobil Corporation, trade name: Vistamaxx 6202) and 50 wt% of an olefin-based resin (manufactured by Mitsui Chemicals, Inc., trade name: TAFMER PN-3560, density=0.866 g/cm³, MFR=6.0 g/10 min) was loaded into A layer in an extrusion machine and a formulation of 25 wt% of an olefin-based resin (manufactured by Exxon Mobil Corporation, trade name: Vistamaxx 3000), 70 wt% of an olefin-based resin (manufactured by Mitsui Chemicals, Inc., trade name: TAFMER PN-3560), and 5 wt% of a white pigment (titanium oxide manufactured by Dupont, trade name: Ti-Pure R103) was loaded into B layer in the extrusion machine to extrude an elastic film (4) having the construction of A layer/B layer/A layer=6.75 um/31.5 µm/6.75 µm in total of 45 um.

Next, 100 parts of a propylene-based elastomer (manufactured by Exxon Mobil Corporation, trade name: Vistamaxx 7050), 517 parts of a propylene/1-butene copolymer (manufactured by REXtac, LLC, trade name: REXTAC RT2788), 520 parts of a tackifier (manufactured by Exxon Mobil Corporation, trade name: Escorez 5400), 150 parts of liquid paraffin (manufactured by Petro yag, trade name: White Oil Pharma Oyster 259), and 13 parts of an antioxidant (manufactured by BASF Japan Co., Ltd., trade name: Irganox 1010) were blended to provide a hot-melt pressure-sensitive adhesive (4).

The resultant elastic film (4) andhot-melt pressure-sensitive adhesive (4) were subjected to the above-mentioned <Forming Conditions>, and a portion (A) in which the hot-melt pressure-sensitive adhesive had been applied onto the whole surface (7 g/m²) with a width of 30 mm was cut at the middle to provide a stretchable laminate (4) having two portions (A1) and (A2), in which the hot-melt pressure-sensitive adhesive had been applied onto the whole surface (7 g/m²) with a width of 15 mm from both ends, and a portion (B), in which the hot-melt pressure-sensitive adhesive had been applied (15 g/m²) between the (A1) and the (A2) with a width of 41 mm in a striped manner (pressure-sensitive adhesive width: 1 mm, interval: 1 mm), on both surfaces of the elastic film.

The results are shown in Table 1 and Table 2.

### [Example 5]

100 wt% of an olefin-based resin (manufactured by Exxon Mobil Corporation, trade name: Vistamaxx 7010) was loaded into A layer in an extrusion machine and a formulation of 45 wt% of an olefin-based resin (manufactured by Exxon Mobil Corporation, trade name: Vistamaxx 3000), 50 wt% of an olefin-based resin (manufactured by Mitsui Chemicals, Inc., trade name: TAFMER PN-3560), and 5 wt% of a white pigment (titanium oxide manufactured by Dupont, trade name: Ti-Pure R103) was loaded into B layer in the extrusion machine to extrude an elastic film (5) having the construction of A layer/B layer/A layer=6.75 µm/31.5 µm/6.75 um in total of 45 µm.

Next, 55 parts of a propylene-based elastomer (manufactured by Exxon Mobil Corporation, trade name: Vistamaxx 7050), 562 parts of a propylene/1-butene copolymer (manufactured by REXtac, LLC, trade name: REXTAC RT2788), 520 parts of a tackifier (manufactured by Exxon Mobil Corporation, trade name: Escorez 5400), 150 parts of liquid paraffin (manufactured by Petro yag, trade name: White Oil Pharma Oyster 259), and 13 parts of an antioxidant (manufactured by BASF Japan Co., Ltd., trade name: Irganox 1010) were blended to provide a hot-melt pressure-sensitive adhesive (5).

The resultant elastic film (5) and hot-meltpressure-sensitive adhesive (5) were subjected to the above-mentioned <Forming Conditions>, and a portion (A) in which the hot-melt pressure-sensitive adhesive had been applied onto the whole surface (7 g/m²) with a width of 30 mm was cut at the middle to provide a stretchable laminate (5) having two portions (A1) and (A2), in which the hot-melt pressure-sensitive adhesive had been applied onto the whole surface (7 g/m²) with a width of 15 mm from both ends, and a portion (B), in which the hot-melt pressure-sensitive adhesive had been applied (15 g/m²) between the (A1) and the (A2) with a width of 41 mm in a striped manner (pressure-sensitive adhesive width: 1 mm, interval: 1 mm), on both surfaces of the elastic film.

The results are shown in Table 1 and Table 2.

### [Example 6]

100 wt% of an olefin-based resin (manufactured by Exxon Mobil Corporation, trade name: Vistamaxx 7010) was loaded into A layer in an extrusion machine and a formulation of 25 wt% of an olefin-based resin (manufactured by Exxon Mobil Corporation, trade name: Vistamaxx 3000), 70 wt% of an olefin-based resin (manufactured by Mitsui Chemicals, Inc., trade name: TAFMER PN-3560), and 5 wt% of a white pigment (titanium oxide manufactured by Dupont, trade name: Ti-Pure R103) was loaded into B layer in the extrusion machine to extrude an elastic film (6) having the construction of A layer/B layer/A layer=6.75 µm/31.5 µm/6.75 µm in total of 45 µm.

Next, 400 parts of a propylene/1-butene copolymer (manufactured by REXtac, LLC, trade name: REXTAC RT2788), 267 parts of a propylene/1-butene copolymer (manufactured by REXtac, LLC, trade name: REXTAC RT2730), 520 parts of a tackifier (manufactured by Exxon Mobil Corporation, trade name: Escorez 5400), 100 parts of liquid paraffin (manufactured by Petro yag, trade name: White Oil Pharma Oyster 259), and 13 parts of an antioxidant (manufactured by BASF Japan Co., Ltd., trade name: Irganox 1010) were blended to provide a hot-melt pressure-sensitive adhesive (6).

The resultant elastic film (6) and hot-melt pressure-sensitive adhesive (6) were subjected to the above-mentioned <Forming Conditions>, and a portion (A) in which the hot-melt pressure-sensitive adhesive had been applied onto the whole surface (7 g/m²) with a width of 30 mm was cut at the middle to provide a stretchable laminate (6) having two portions (A1) and (A2), in which the hot-melt pressure-sensitive adhesive had been applied onto the whole surface (7 g/m²) with a width of 15 mm from both ends, and a portion (B), in which the hot-melt pressure-sensitive adhesive had been applied (15 g/m²) between the (A1) and the (A2) with a width of 41 mm in a striped manner (pressure-sensitive adhesive width: 1 mm, interval: 1 mm), on both surfaces of the elastic film.

The results are shown in Table 1 and Table 2.

### [Example 7] - Comparative example

100 wt% of an olefin-based resin (manufactured by Exxon Mobil Corporation, trade name: Vistamaxx 7010) was loaded into A layer in an extrusion machine and a formulation of 50 wt% of an SIS-based resin (manufactured by Zeon Corporation, trade name: Quintac 3399), 45 wt% of an SBS-based resin (manufactured by Kraton Performance Polymers Inc., trade name: Kraton D1191), and 5 wt% of a white pigment (titanium oxide manufactured by Dupont, trade name: Ti-Pure R103) was loaded into B layer in the extrusion machine to extrude an elastic film (7) having the construction of A layer/B layer/A layer=6.75 µm/31.5 µm/6.75 µm in total of 45 um.

Next, 300 parts of a propylene/1-butene copolymer (manufactured by REXtac, LLC, trade name: REXTAC RT2788), 267 parts of a propylene/1-butene copolymer (manufactured by REXtac, LLC, trade name: REXTAC RT2730), 100 parts of a propylene-based elastomer (manufactured by Exxon Mobil Corporation, trade name: Vistamaxx 7050), 520 parts of a tackifier (manufactured by Exxon Mobil Corporation, trade name: Escorez 5400), 100 parts of liquid paraffin (manufactured by Petro yag, trade name: White Oil Pharma Oyster 259), and 13 parts of an antioxidant (manufactured by BASF Japan Co., Ltd. , trade name: Irganox1010) were blended to provide a hot-melt pressure-sensitive adhesive (7).

The resultant elastic film (7) and hot-melt pressure-sensitive adhesive (7) were subjected to the above-mentioned <Forming Conditions>, and a portion (A) in which the hot-melt pressure-sensitive adhesive had been applied onto the whole surface (7 g/m²) with a width of 30 mm was cut at the middle to provide a stretchable laminate (7) having two portions (A1) and (A2), in which the hot-melt pressure-sensitive adhesive had been applied onto the whole surface (7 g/m²) with a width of 15 mm from both ends, and a portion (B), in which the hot-melt pressure-sensitive adhesive had been applied (15 g/m²) between the (A1) and the (A2) with a width of 41 mm in a striped manner (pressure-sensitive adhesive width: 1 mm, interval: 1 mm), on both surfaces of the elastic film.

The results are shown in Table 1 and Table 2.

### [Example 8] - Comparative example

100 wt% of an olefin-based resin (manufactured by Exxon Mobil Corporation, trade name: Vistamaxx 7010) was loaded into A layer in an extrusion machine and a formulation of 25 wt% of an SIS-based resin (manufactured by Zeon Corporation, trade name: Quintac 3399), 70 wt% of an SBS-based resin (manufactured by Kraton Performance Polymers Inc., trade name: Kraton D1191), and 5 wt% of a white pigment (titanium oxide manufactured by Dupont, trade name: Ti-Pure R103) was loaded into B layer in the extrusion machine to extrude an elastic film (8) having the construction of A layer/B layer/A layer=6.75 µm/31.5 µm/6.75 um in total of 45 um.

Next, 300 parts of a propylene/1-butene copolymer (manufactured by REXtac, LLC, trade name: REXTAC RT2788), 217 parts of a propylene/1-butene copolymer (manufactured by REXtac, LLC, trade name: REXTAC RT2730), 100 parts of a propylene-based elastomer (manufactured by Exxon Mobil Corporation, trade name: Vistamaxx 7050), 520 parts of a tackifier (manufactured by Exxon Mobil Corporation, trade name: Escorez 5400), 150 parts of liquid paraffin (manufactured by Petro yag, trade name: White Oil Pharma Oyster 259), and 13 parts of an antioxidant (manufactured by BASF Japan Co. , Ltd. , tradename: Irganox1010) were blended to provide a hot -melt pressure-sensitive adhesive (8).

The resultant elastic film (8) andhot-melt pressure-sensitive adhesive (8) were subjected to the above-mentioned <Forming Conditions>, and a portion (A) in which the hot-melt pressure-sensitive adhesive had been applied onto the whole surface (7 g/m²) with a width of 30 mm was cut at the middle to provide a stretchable laminate (8) having two portions (A1) and (A2), in which the hot-melt pressure-sensitive adhesive had been applied onto the whole surface (7 g/m²) with a width of 15 mm from both ends, and a portion (B), in which the hot-melt pressure-sensitive adhesive had been applied (15 g/m²) between the (A1) and the (A2) with a width of 41 mm in a striped manner (pressure-sensitive adhesive width: 1 mm, interval: 1 mm), on both surfaces of the elastic film.

The results are shown in Table 1 and Table 2.

### [Comparative Example 1]

A stretchable laminate (C1) was obtained in the same manner as in Example 1 except that a hot-melt pressure-sensitive adhesive (C1) obtained by blending 213 parts of an SIS-based resin (manufactured by Kraton Performance Polymers Inc., trade name: Kraton D1165 PT), 619 parts of a tackifier (manufactured by Kolon Industries, Inc., trade name: SUKOREZ SU-100S), 84 parts of liquid paraffin (manufactured by Petro yag, trade name: White Oil Pharma Oyster 259), and 10 parts of an antioxidant (manufactured by BASF Japan Co. , Ltd., trade name: Irganox 1010) was used instead of the hot-melt pressure-sensitive adhesive (1).

The results are shown in Table 3 and Table 4.

### [Comparative Example 2]

A stretchable laminate (C2) was obtained in the same manner as in Example 2 except that a hot-melt pressure-sensitive adhesive (C2) obtained by blending 25 parts of an SIS-based resin (manufactured by Kraton Performance Polymers Inc., trade name: Kraton D1119), 189 parts of an SIS-based resin (manufactured by Kraton Performance Polymers Inc., trade name: Kraton D1161), 619 parts of a tackifier (manufactured by Kolon Industries, Inc., trade name: SUKOREZ SU-100S), 34 parts of liquid paraffin (manufactured by Petro yag, trade name: White Oil Pharma Oyster 259), 9 parts of an antioxidant (manufactured by BASF Japan Co., Ltd., trade name: Irganox 1010), and 50 parts of polyisobutylene (PIB manufactured by Ineos Group Limited, trade name: Indopol H-300) was used instead of the hot-melt pressure-sensitive adhesive (2).

The results are shown in Table 3 and Table 4.

### [Comparative Example 3]

A stretchable laminate (C3) was obtained in the same manner as in Example 3 except that a hot-melt pressure-sensitive adhesive (C3) obtained by blending 100 parts of an SIS-based resin (manufactured by Kraton Performance Polymers Inc., trade name: Kraton D1160 BT), 113 parts of an SIS-based resin (manufactured by Kraton Performance Polymers Inc., trade name: Kraton D1161), 619 parts of a tackifier (manufactured by Eastman Chemical Company, trade name: Eastotac H-100W), 34 parts of liquid paraffin (manufactured by Petro yag, trade name: White Oil Pharma Oyster 259), 9 parts of an antioxidant (manufactured by BASF Japan Co., Ltd., trade name: Irganox 1010), and 50 parts of polyisobutylene (PIB manufactured by Ineos Group Limited, trade name: Indopol H-300) was used instead of the hot-melt pressure-sensitive adhesive (3) .

The results are shown in Table 3 and Table 4.

### [Comparative Example 4]

A stretchable laminate (C4) was obtained in the same manner as in Example 4 except that a hot-melt pressure-sensitive adhesive (C4) obtained by blending 100 parts of an SIS-based resin (manufactured by Kraton Performance Polymers Inc., trade name: Kraton D1160 BT), 113 parts of an SIS-based resin (manufactured by Kraton Performance Polymers Inc., trade name: Kraton D1162), 619 parts of a tackifier (manufactured by Eastman Chemical Company, trade name: Eastotac H-100W), 34 parts of liquid paraffin (manufactured by Petro yag, trade name: White Oil Pharma Oyster 259), 9 parts of an antioxidant (manufactured by BASF Japan Co., Ltd., trade name: Irganox 1010), and 50 parts of polyisobutylene (PIB manufactured by Ineos Group Limited, trade name: Indopol H-300) was used instead of the hot-melt pressure-sensitive adhesive (4) .

The results are shown in Table 3 and Table 4.

### [Comparative Example 5]

A stretchable laminate (C5) was obtained in the same manner as in Example 5 except that a hot-melt pressure-sensitive adhesive (C5) obtained by blending 100 parts of an SIS-based resin (manufactured by Kraton Performance Polymers Inc., trade name: Kraton D1160 BT), 113 parts of an SIS-based resin (manufactured by Kraton Performance Polymers Inc., trade name: Kraton D1162), 619 parts of a tackifier (manufactured by Eastman Chemical Company, trade name: Eastotac H-100W), 120 parts of liquid paraffin (manufactured by Petro yag, trade name: White Oil Pharma Oyster 259), and 9 parts of an antioxidant (manufactured by BASF Japan Co., Ltd., trade name: Irganox1010) was used instead of the hot-melt pressure-sensitive adhesive (5).

The results are shown in Table 3 and Table 4.

### [Comparative Example 6]

A stretchable laminate (C6) was obtained in the same manner as in Example 6 except that a hot-melt pressure-sensitive adhesive (C6) obtained by blending 100 parts of an SIS-based resin (manufactured by Kraton Performance Polymers Inc., trade name: Kraton D1160 BT), 113 parts of an SIS-based resin (manufactured by Kraton Performance Polymers Inc., trade name: Kraton D1165 PT), 619 parts of a tackifier (manufactured by Eastman Chemical Company, trade name: Eastotac H-100W), 120 parts of liquid paraffin (manufactured by Petro yag, trade name: White Oil Pharma Oyster 259), and 9 parts of an antioxidant (manufactured by BASF Japan Co. , Ltd., trade name: Irganox 1010) was used instead of the hot-melt pressure-sensitive adhesive (6).

The results are shown in Table 3 and Table 4.

### [Comparative Example 7]

A stretchable laminate (C7) was obtained in the same manner as in Example 7 except that a hot-melt pressure-sensitive adhesive (C7) obtained by blending 100 parts of an SIS-based resin (manufactured by Kraton Performance Polymers Inc., trade name: Kraton D1160 BT), 113 parts of an SIS-based resin (manufactured by Kraton Performance Polymers Inc., trade name: Kraton D1161), 619 parts of a tackifier (manufactured by Eastman Chemical Company, trade name: Eastotac H-100W), 120 parts of liquid paraffin (manufactured by Petro yag, trade name: White Oil Pharma Oyster 259), and 9 parts of an antioxidant (manufactured by BASF Japan Co., Ltd., trade name: Irganox 1010) was used instead of the hot-melt pressure-sensitive adhesive (7).

The results are shown in Table 3 and Table 4.

### [Comparative Example 8]

A stretchable laminate (C8) was obtained in the same manner as in Example 8 except that a hot-melt pressure-sensitive adhesive (C8) obtained by blending 100 parts of an SIS-based resin (manufactured by Kraton Performance Polymers Inc., trade name: Kraton D1160 BT), 113 parts of an SIS-based resin (manufactured by Kraton Performance Polymers Inc., trade name: Kraton D1161), 619 parts of a tackifier (manufactured by Arakawa Chemical Industries, Ltd., trade name: ARKON P-140), 120 parts of liquid paraffin (manufactured by Petro yag, trade name: White Oil Pharma Oyster 259), and 9 parts of an antioxidant (manufactured by BASF Japan Co., Ltd., trade name: Irganox1010) was used instead of the hot-melt pressure-sensitive adhesive (8).

The results are shown in Table 3 and Table 4.

**[Table 1]**

| | | ' Example | | | | | | | |
|---|---|---|---|---|---|---|---|---|---|
| | | 1 | 2 | 3 | 4 | 5 | 6 | 7 | 8 |
| A layer resin (1) | | Vistamaxx 6202 | Vistamaxx 6202 | Vistamaxx 6202 | Vistamaxx 6202 | Vistamaxx 7010 | Vistamaxx 7010 | Vistamaxx 7010 | Vistamaxx 7010 |
| A layer resin (2) | | - | - | Tafmer PN-3560 | Tafmer PN-3560 | | | | |
| B layer resin (1) | | Vistamaxx 6202 | Vistamaxx 6202 | Vistamaxx 3000 | Vistamaxx 3000 | Vistamaxx 3000 | Vistamaxx 3000 | Quintac 3399 | Quintac 3399 |
| B layer resin (2) | | Tafmer PN-3560 | Tafmer PN-3560 | Tafmer PN-3560 | Tafmer PN-3560 | Tafmer PN-3560 | Tafmer PN-3560 | Kraton D1191 | Kraton D1191 |
| B layer resin (3) | | Ti02 | Ti02 | TiO2 | TiO2 | TiO2 | TiO2 | Ti02 | TiO2 |
| A layer formulation (1)/(2) | | 100/0 | 100/0 | 50/50 | 50/50 | 100/0 | 100/0 | 100/0 | 100/0 |
| B layer formulation (1)/(2)/(3) | | 65/30/5 | 65/30/5 | 45/50/5 | 25/75/5 | 45/50/5 | 25/75/5 | 50/45/5 | 25/75/5 |
| A/B/A thickness | µm | 6.75/31.5/6. 75 | 6.75/31.5/6. 75 | 6.75/31.5/6. 75 | 6.75/31.5/6. 75 | 6.75/31.5/6. 75 | 6.75/31.5/6. 75 | 6.75/31.5/6. 75 | 6.75/31.5/6. 75 |
| Total thickness of elastic film | µm | 45 | 45 | 45 | 45 | 45 | 45 | 45 | 45 |
| Kind of hot-melt pressure-sens itive adhesive | | Olefin 1 | Olefin 2 | Olefin 3 | Olefin 4 | Olefin 5 | Olefin 6 | Olefin 7 | Olefin 8 |
| Type of non-woven fabric | | Carded 24 g/m² | Carded 24 g/m² | Carded 24 g/m² | Carded 24 g/m² | Carded 24 g/m² | Carded 24 g/m² | Carded 24 g/m² | Carded 24 g/m² |
| PP non-woven fabric adhesive strength (90° peeling) | g/2 5 mm | 423 | 350 | 356 | 367 | 326 | 246 | 388 | 343 |
| 40°C retention ability test Presence/abse nce of delamination | OK/ NG | OK | OK | OK | OK | OK | OK | OK | OK |

**[Table 2]**

| Olefin 1 | | Olefin 2 | | Olefin 3 | | Olefin 4 | |
|---|---|---|---|---|---|---|---|
| Raw materials | Composition (kg) | Raw materials | Composition (kg) | Raw materials | Composition (kg) | Raw materials | Composition (kg) |
| REXTAC RT2788 | 667 | Vistamaxx 6202 | 100 | Vistamaxx 7050 | 100 | Vistamaxx 7050 | 100 |
| SUKOREZ SU-100 S | 520 | REXTAC RT2788 | 517 | REXTAC RT2730 | 517 | REXTAC RT2788 | 517 |
| White Oil Pharma | 100 | Escorez 5400 | 520 | Escorez 5400 | 520 | Escorez 5400 | 520 |
| Irganox 1010 | 13 | White Oil Pharma | 150 | White Oil Pharma | 100 | White Oil Pharma | 150 |
| Total | 1,300 | Irganox 1010 | 13 | Irganox 1010 | 13 | Irganox 1010 | 13 |
| | | Total | 1,300 | Total | 1,250 | Total | 1,300 |

| Olefin 5 | | Olefin 6 | | Olefin 7 | | Olefin 8 | |
|---|---|---|---|---|---|---|---|
| Raw materials | Composition (kg) | Raw materials | Composition (kg) | Raw materials | Composition (kg) | Raw materials | Composition (kg) |
| Vistamaxx 7050 | 55 | REXTAC RT2788 | 400 | REXTAC RT2788 | 300 | REXTAC RT2788 | 300 |
| REXTAC RT2788 | 562 | REXTAC RT2730 | 267 | REXTAC RT2730 | 267 | REXTAC RT2730 | 217 |
| Escorez 5400 | 520 | Escorez 5400 | 520 | Vistamaxx 7050 | 100 | Vistamaxx 7050 | 100 |
| White Oil Pharma | 150 | White Oil Pharma | 100 | Escorez 5400 | 520 | Escorez 5400 | 520 |
| Irganox 1010 | 13 | Irganox 1010 | 13 | White Oil Pharma | 100 | White Oil Pharma | 150 |
| Total | 1,300 | Total | 1,300 | Irganox 1010 | 13 | Irganox 1010 | 13 |
| | | | | Total | 1,300 | Total | 1,300 |

**[Table 3]**

| | | Comparative Example | | | | | | | |
|---|---|---|---|---|---|---|---|---|---|
| | | 1 | 2 | 3 | 4 | 5 | 6 | 7 | 8 |
| A layer resin (1) | | Vistamaxx 6202 | ← | ← | ← | Vistamaxx 7010 | Vistamaxx 7010 | Vistamaxx 7010 | Vistamaxx 7010 |
| A layer resin (2) | | - | - | Tafmer PN-3560 | ← | - | - | - | - |
| B layer resin (1) | | Vistamaxx 6202 | - | Vistamaxx 3000 | ← | ← | ← | Quintac 3399 | ← |
| B layer resin (2) | | Tafmer PN-3560 | ← | | ← | ← | ← | Kraton D1191 | - |
| B layer resin (3) | | TiO2 | ← | | | | | | |
| A layer formulation (1) / (2) | | 100/0 | 100/0 | 50/50 | 50/50 | 100/0 | 100/0 | 100/0 | 100/0 |
| B layer formulation (1)/(2)/(3) | | 65/30/5 | ← | 45/50/5 | 25/75/5 | 45/50/5 | 25/75/5 | 50/45/5 | 25/75/5 |
| A/B/A thickness | µm | 6.75/31.5/6.7 5 | 6.75/31.5/6.7 5 | 6.75/31.5/6.7 5 | 6.75/31.5/6.7 5 | 6.75/31.5/6.7 5 | 6.75/31.5/6.7 5 | 6.75/31.5/6.7 5 | 6.75/31.5/6.7 5 |
| Total thickness of elastic film | um | 45 | 45 | 45 | 45 | 45 | 45 | 45 | 45 |
| Kind of hot-melt pressure-sensiti ve adhesive | | Styrene 1 | Styrene 2 | Styrene 3 | Styrene 4 | Styrene 5 | Styrene 6 | Styrene 7 | Styrene 8 |
| Type of non-woven fabric | | Carded 24 g/m² | Carded 24 g/m² | Carded 24 g/m² | Carded 24 g/m² | Carded 24 g/m² | Carded 24 g/m² | Carded 24 g/m² | Carded 24 g/m² |
| PP non-woven fabric adhesive strength (90° peeling) | g/2 5 mm | 71 | 77 | 106 | 25 | 100 | 185 | 174 | 6 |
| 40°C retention ability test Presence/absence of delamination | OK/ NG | NG | NG | NG | NG | NG | NG | NG | NG |

**[Table 4]**

| Styrene 1 | | Styrene 2 | | Styrene 3 | | Styrene 4 | |
|---|---|---|---|---|---|---|---|
| Raw materials | Composition (kg) | Raw materials | Composition (kg) | Raw materials | Composition (kg) | Raw materials | Composition (kg) |
| Kraton D1165 PT | 213 | Kraton D1119 | 25 | Kraton D1160 BT | 100 | Kraton D1160 BT | 100 |
| Sukorez SU-100 | 619 | Kraton D1161 | 189 | Kraton D1161 | 113 | Kraton D1162 | 113 |
| Irganox 1010 | 10 | Sukorez SU-100 | 619 | Eastotac H-100W | 619 | Eastotac H-100W | 619 |
| White Oil Pharma | 84 | Irganox 1010 | 9 | Irganox 1010 | 9 | Irganox 1010 | 9 |
| Total | 926 | White Oil Pharma | 34 | White Oil Pharma | 34 | White Oil Pharma | 34 |
| | | PIB Indopol H 300 | 50 | PIB Indopol H 300 | 50 | PIB Indopol H 300 | 50 |
| | | Total | 926 | Total | 925 | Total | 925 |

| Styrene 5 | | Styrene 6 | | Styrene 7 | | Styrene 8 | |
|---|---|---|---|---|---|---|---|
| Raw materials | Composition (kg) | Raw materials | Composition (kg) | Raw materials | Composition (kg) | Raw materials | Composition (kg) |
| Kraton D1160 BT | 100 | Kraton D1160 BT | 100 | Kraton D1160 BT | 100 | Kraton D1160 BT | 100 |
| Kraton D1162 | 113 | Kraton D1165 PT | 113 | Kraton D1161 | 113 | Kraton D1161 | 113 |
| Eastotac H-100W | 619 | Eastotac H-100W | 619 | Eastotac H-100W | 619 | ARKON P-140 | 619 |
| Irganox 1010 | 9 | Irganox 1010 | 9 | Irganox 1010 | 9 | Irganox 1010 | 9 |
| White Oil Pharma | 120 | White Oil Pharma | 120 | White Oil Pharma | 120 | White Oil Pharma | 120 |
| Total | 961 | Total | 961 | Total | 961 | Total | 961 |

The stretchable laminate of the present invention may be used in any appropriate article in which the effects of the present invention can be effectively utilized. That is, the article of the present invention includes the stretchable laminate of the present invention. A typical example of such article is a sanitary article. Examples of such sanitary article include a diaper (particularly, an ear portion of a disposable diaper), a supporter, and a mask.

### Reference Signs List

- **100**: stretchable laminate
- **10**: elastomer layer
- **20**: non-woven fabric layer
- **20a**: non-woven fabric layer
- **20b**: non-woven fabric layer
- **30**: hot-melt pressure-sensitive adhesive
- **30a**: hot-melt pressure-sensitive adhesive
- **30b**: hot-melt pressure-sensitive adhesive

## Claims

1. A stretchable laminate (100), comprising:
an elastomer layer (10), wherein the elastomer layer (10) comprises an olefin-based elastomer as a main component, wherein the content of the olefin-based elastomer serving as a main component in the elastomer layer is from 50 wt% to 100 wt%, wherein the olefin-based elastomer is propylene-based elastomer; and
a non-woven fabric layer (20) arranged on at least one side of the elastomer layer (10), wherein the non-woven fabric forming the non-woven fabric layer (20) contains polyolefin fibers, wherein the polyolefin fibers are polypropyene fibers,
the stretchable laminate (100) further comprising a hot-melt pressure-sensitive adhesive (30) between the elastomer layer (10) and the non-woven fabric layer (20),
the hot-melt pressure-sensitive adhesive (30) containing an olefin component, wherein the olefin component comprises an olefin-based polymer, wherein the olefin-based polymer comprises a 1-butene copolymer as an essential component, wherein a content of the 1-butene copolymer in the hot-melt pressure-sensitive adhesive (30) is from 35 wt% to 60 wt%.

2. The stretchable laminate (100) according to claim 1, wherein the olefin-based polymer further comprises an olefin-based elastomer.

3. The stretchable laminate (100) according to claim 2, wherein the olefin-based elastomer is an α-olefin-based elastomer.

4. The stretchable laminate (100) according to claim 3, wherein the α-olefin-based elastomer comprises at least one kind selected from an ethylene-based elastomer, a propylene-based elastomer, and a 1-butene-based elastomer.

5. The stretchable laminate (100) according to claim 1, wherein the hot-melt pressure-sensitive adhesive (30) comprises a tackifier.

6. The stretchable laminate (100) according to claim 5, wherein a content of the tackifier in the hot-melt pressure-sensitive adhesive (30) is from 35 wt% to 55 wt%.

7. An article, comprising the stretchable laminate (100) of claim 1.

## Patentansprüche

1. Dehnbares Laminat (100), umfassend:
eine Elastomerschicht (10), wobei die Elastomerschicht (10) ein Elastomer auf Olefinbasis als eine Hauptkomponente umfasst, wobei der Gehalt des Elastomers auf Olefinbasis, das als eine Hauptkomponente in der Elastomerschicht dient, von 50 Gew.-% bis 100 Gew.-% beträgt, wobei das Elastomer auf Olefinbasis ein Elastomer auf Propylenbasis ist; und
eine Vliesstoffschicht (20), die auf mindestens einer Seite der Elastomerschicht (10) angeordnet ist, wobei der Vliesstoff, der die Vliesstoffschicht (20) bildet, Polyolefinfasern enthält, wobei die Polyolefinfasern Polypropylenfasern sind,
wobei das dehnbare Laminat (100) weiter einen druckempfindlichen Heißschmelzklebstoff (30) zwischen der Elastomerschicht (10) und der Vliesstoffschicht (20) umfasst,
wobei der druckempfindliche Heißschmelzklebstoff (30) einen Olefinbestandteil enthält, wobei der Olefinbestandteil ein Polymer auf Olefinbasis umfasst, wobei das Polymer auf Olefinbasis ein 1-Buten-Copolymer als einen wesentlichen Bestandteil umfasst, wobei ein Gehalt des 1-Buten-Copolymers in dem druckempfindlichen Heißschmelzklebstoff (30) von 35 Gew.-% bis 60 Gew.-% beträgt.

2. Dehnbares Laminat (100) nach Anspruch 1, wobei das Polymer auf Olefinbasis weiter ein Elastomer auf Olefinbasis umfasst.

3. Dehnbares Laminat (100) nach Anspruch 2, wobei das Elastomer auf Olefinbasis ein Elastomer auf α-Olefinbasis ist.

4. Dehnbares Laminat (100) nach Anspruch 3, wobei das Elastomer auf α-Olefinbasis mindestens eine Art umfasst, ausgewählt aus einem Elastomer auf Ethylenbasis, einem Elastomer auf Propylenbasis und einem Elastomer auf 1-Butenbasis.

5. Dehnbares Laminat (100) nach Anspruch 1, wobei der druckempfindliche Heißschmelzklebstoff (30) einen Klebrigmacher umfasst.

6. Dehnbares Laminat (100) nach Anspruch 5, wobei der Gehalt des Klebrigmachers in dem druckempfindlichen Heißschmelzklebstoff (30) von 35 Gew.-% bis 55 Gew.-% beträgt.

7. Gegenstand, umfassend das dehnbare Laminat (100) nach Anspruch 1.

## Revendications

1. Stratifié extensible (100), comprenant :
une couche d'élastomère (10), dans lequel la couche d'élastomère (10) comprend un élastomère à base d'oléfine en tant que composant principal, dans lequel la teneur en élastomère à base d'oléfine servant de composant principal dans la couche d'élastomère va de 50% en poids à 100% en poids, dans lequel l'élastomère à base d'oléfine est un élastomère à base de propylène; et
une couche de tissu non tissé (20) disposée sur au moins un côté de la couche d'élastomère (10), dans lequel le tissu non tissé formant la couche de tissu non tissé (20) contient des fibres de polyoléfine, dans lequel les fibres de polyoléfine sont des fibres de polypropylène,
le stratifié extensible (100) comprenant en outre un adhésif sensible à la pression thermofusible (30) entre la couche d'élastomère (10) et la couche de tissu non tissé (20),
l'adhésif sensible à la pression thermofusible (30) contenant un composant d'oléfine, dans lequel le composant d'oléfine comprend un polymère à base d'oléfine, dans lequel le polymère à base d'oléfine comprend un copolymère de 1-butène en tant que composant essentiel, dans lequel la teneur en copolymère de 1-butène dans l'adhésif sensible à la pression thermofusible (30) va de 35% en poids à 60% en poids.

2. Stratifié extensible (100) selon la revendication 1, dans lequel le polymère à base d'oléfine comprend en outre un élastomère à base d'oléfine.

3. Stratifié extensible (100) selon la revendication 2, dans lequel l'élastomère à base d'oléfine est un élastomère à base d'α-oléfine.

4. Stratifié extensible (100) selon la revendication 3, dans lequel l'élastomère à base d'α-oléfine comprend au moins une sorte sélectionnée parmi un élastomère à base d'éthylène, un élastomère à base de propylène et un élastomère à base de 1-butène.

5. Stratifié extensible (100) selon la revendication 1, dans lequel l'adhésif sensible à la pression thermofusible (30) comprend un agent tackifiant.

6. Stratifié extensible (100) selon la revendication 5, dans lequel la teneur en agent tackifiant dans l'adhésif sensible à la pression thermofusible (30) va de 35% en poids à 55% en poids.

7. Article comprenant le stratifié extensible (100) selon la revendication 1.
